(19) Europäisches Patentamt

European Patent Office

Office européen des brevets

(11) EP 0 779 071 B1

## (12) FASCICULE DE BREVET EUROPEEN

(45) Date de publication et mention
de la délivrance du brevet:
**15.12.1999 Bulletin 1999/50**

(51) Int. Cl.$^6$: **A61K 9/113**, A61K 7/00

(21) Numéro de dépôt: **96402492.1**

(22) Date de dépôt: **20.11.1996**

(54) **Emulsion E/H/E stable contenant un actif cosmétique et/ou dermatologique sensible à l'eau**

Stabile W/Ö/W-Emulsion, die einen wasserempfindlichen kosmetischen und/oder dermatologischen
Wirkstoff enthält

Stable w/o/w emulsion containing a water-sensitive cosmetic and/or dermatologic agent

(84) Etats contractants désignés:
**AT BE CH DE ES FR GB IT LI NL SE**

(30) Priorité: **15.12.1995 FR 9514940**

(43) Date de publication de la demande:
**18.06.1997 Bulletin 1997/25**

(73) Titulaire: **L'OREAL**
**75008 Paris (FR)**

(72) Inventeurs:
• **Afriat, Isabelle**
**75014 Paris (FR)**

• **Gagnebien, Didier**
**92320 Chatillon (FR)**

(74) Mandataire:
**Tezier Herman, Béatrice**
**L' OREAL,**
**D.P.I.,**
**6, rue Bertrand Sincholle**
**92585 Clichy Cédex (FR)**

(56) Documents cités:
**EP-A- 0 278 103        EP-A- 0 755 673**
**FR-A- 2 693 466        FR-A- 2 693 733**

## Description

La présente invention se rapporte à un milieu stable susceptible de contenir un actif sensible à l'eau, utilisable en particulier dans les domaines cosmétique et/ou dermatologique pour nettoyer et/ou soigner et/ou protéger la peau et/ou les muqueuses et/ou les fibres kératiniques.

Il est connu d'introduire dans les compositions cosmétiques et/ou dermatologiques des actifs en vue d'apporter des traitements spécifiques à la peau et/ou aux cheveux, par exemple pour nettoyer la peau, pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire, pour traiter la séborrhée des cheveux.

Par exemple, il est connu d'introduire dans les compositions cosmétiques des enzymes, et notamment des protéases utilisées pour leurs propriétés protéolytiques. Ces enzymes sont recherchées dans le domaine cosmétique pour leur pouvoir lissant et nettoyant, et leur aptitude à éliminer les cellules mortes de la peau.

Malheureusement, certains actifs, et en particulier ceux cités ci-dessus, présentent l'inconvénient d'être instables en milieu aqueux et d'être facilement dégradés ou modifiés sous l'influence de l'eau. Ils perdent ainsi rapidement de leur activité au cours du temps et cette instabilité va à l'encontre de l'efficacité recherchée.

Aussi, différents moyens ont été envisagés pour pallier à cet inconvénient. En particulier, il a été envisagé de mettre un actif, notamment une enzyme dans une composition pulvérulente (voir le document JP-A-63-130514). D'ailleurs, la plupart des produits de nettoyage de la peau contenant une enzyme se présentent sous cette forme. Il a été aussi envisagé d'utiliser ces actifs, et notamment les enzymes, sous forme immobilisée sur des supports polymériques (voir le document JP-A-61-207499) ou dans des microcapsules (voir le document JP-A-61-254244). Malheureusement, certains de ces moyens nécessitent une mise en oeuvre particulière, ce qui accroît le coût et le temps de préparation de la composition.

Une autre solution consiste à les incorporer dans un milieu liquide anhydre (voir le document US-A-5322683). Malheureusement, cette solution limite la forme galénique de la composition et ne permet pas l'incorporation d'actifs hydrophiles.

Il subsiste donc le besoin d'un milieu stable susceptible de contenir des actifs cosmétiques et/ou dermatologiques hydrophiles et/ou lipophiles sensibles à l'eau, dans lequel ces derniers conserveraient toutes leurs propriétés et donc leur efficacité au cours du temps.

La demanderesse a maintenant trouvé de manière inattendue qu'une composition cosmétique et/ou dermatologique sous forme d'une émulsion triple eau/huile/eau (E/H/E) dont une des phases aqueuses a une valeur d'activité en eau, inférieure ou égale à 0,85, était susceptible de maintenir l'activité d'un actif sensible à l'eau et d'éviter la dégradation de l'actif, comme revendiqué dans revendication 1.

Selon un mode particulier de réalisation de l'invention, la valeur d'activité en eau inférieure ou égale à 0,85 est obtenue par incorporation d'une quantité efficace de polyol. On entend par quantité efficace une quantité suffisante de polyol pour obtenir une faible valeur d'activité en eau, c'est-à-dire une valeur d'activité en eau, inférieure ou égale à 0,85.

Par ailleurs, selon un mode préféré de réalisation de l'invention, la phase huileuse de l'émulsion triple selon l'invention contient au moins une huile siliconée et un émulsionnant siliconé.

De façon avantageuse, cette émulsion peut être utilisée comme support d'actif sensible à l'eau. Celui-ci est introduit dans la phase aqueuse présentant une faible valeur d'activité en eau. Aussi, l'invention a aussi pour objet une composition, caractérisée en ce qu'elle contient une émulsion telle que définie ci-dessus et au moins un actif à action topique sensible à l'eau, contenu dans la phase aqueuse présentant une valeur d'activité en eau, inférieure ou égale à 0,85.

Certes, il est connu d'utiliser dans les domaines cosmétique et dermatologique des émulsions eau/huile/eau dont l'une des phases aqueuses contient des polyols. Ainsi, le document WO-A-94/1073 divulgue des compositions de ce type, mais il n'enseigne pas l'introduction d'une grande quantité de polyol, notamment en vue de stabiliser les actifs sensibles à l'eau. En outre, il n'enseigne ni ne suggère d'avoir une phase aqueuse ayant une faible activité en eau.

Par ailleurs, il est connu que la teneur en eau peut avoir une influence sur la stabilité des actifs sensibles à l'eau, mais il n'a jamais été décrit ni suggéré que la seule présence de polyol puisse éviter la dégradation de tels actifs. Ainsi, le document de D. Tzanos (Behavior of enzymes by controlling the medium water activity ; Riv. Ital. Essenze, Profumi, Piante Off., Aromi, Saponi, Cosmet., Aerosol, 1977, vol.59, n°5, pages 208-211) incite l'homme du métier à utiliser des tensioactifs pour la stabilisation des enzymes en milieu aqueux ou à fixer les enzymes sur un support poreux. En revanche, il écarte l'homme du métier d'utiliser des glycols.

Par ailleurs, le document US-A-5356800 décrit un procédé de stabilisation des enzymes consistant à utiliser un mélange comprenant un alcool ou un glycol, une alkyldiamine oxyéthylénée et un oxyde d'amine. Selon ce document, la stabilisation des enzymes ne peut être obtenue qu'en utilisant le mélange décrit.

En outre, le document JP-A-01-283213 décrit une composition de nettoyage contenant une enzyme et un polyol. Selon ce document, l'activité enzymatique est stabilisée par addition d'une protéine telle que le collagène, l'élastine ou l'albumine.

Or, il a été maintenant trouvé que l'on peut éviter la dégradation de tels actifs en les introduisant dans l'émulsion E/H/E selon l'invention.

L'émulsion triple selon l'invention a l'avantage non seulement de préserver l'activité des actifs sensibles à l'eau, mais aussi de véhiculer néanmoins une quantité suffisante d'eau du fait de la présence d'eau dans la phase aqueuse n'ayant pas une activité en eau réduite. Cette présence d'eau permet à la fois un apport d'eau à la peau et une activation de l'enzyme au moment de l'application sur la peau.

Selon une forme préférée de réalisation de l'invention, la quantité du ou des polyols doit être telle que la valeur d'activité en eau de l'une des phases aqueuses de l'émulsion est inférieure ou égale à 0,75.

L'activité en eau $a_w$ d'un milieu contenant de l'eau est le rapport de la pression de vapeur d'eau du milieu《 $P_{H2O}$ milieu 》 et de la pression de vapeur de l'eau pure《 $P_{H2O}$ pur 》 à la même température. Elle peut être exprimée aussi comme le rapport du nombre de molécules d'eau《 $N_{H2O}$ 》 sur le nombre de molécules totales《 $N_{H2O} + N_{corps\ dissous}$ 》, qui tient compte de celles des corps dissous《 $N_{corps\ dissous}$ 》.

Elle est donnée par les formules suivantes :

$$a_w = \frac{P_{H2O}\ milieu}{P_{H2O}\ pur} = \frac{N_{H2O}}{N_{H2O} + N_{corps\ dissous}}$$

On peut utiliser différentes méthodes pour mesurer l'activité en eau. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable de l'activité en eau.

Dans l'émulsion selon l'invention, l'une ou l'autre des phases aqueuses (interne ou externe) peut présenter une faible activité en eau, mais il s'agit de préférence de la phase aqueuse externe continue.

Pour avoir une telle activité en eau inférieure à 0,85, la quantité de polyol dans la phase considérée doit aller de 35 à 90 % en poids, et mieux de 60 à 85 % en poids par rapport au poids total de la phase aqueuse à faible activité en eau, c'est-à-dire être supérieure à 30 % en poids par rapport au poids total de l'émulsion, et de préférence aller de 35 à 70 % en poids par rapport au poids total de l'émulsion.

Le polyol utilisé selon l'invention peut être notamment choisi parmi la glycérine et les glycols, en particulier le propylène glycol et les polyéthylène glycols.

Selon un mode de réalisation préféré de l'invention, le ou les polyols se trouvent totalement ou partiellement sous forme complexée avec un polymère acrylique ou méthacrylique. Le polymère peut également comprendre de l'eau liée, c'est-à-dire être complexé avec un mélange d'eau et de polyol(s).

On entend par polymère acrylique ou méthacrylique un homopolymère ou un copolymère d'acide acrylique ou méthacrylique ou un homopolymère ou un copolymère d'un dérivé d'acide acrylique ou méthacrylique.

La quantité de tels polymères avec le ou les polyols et éventuellement l'eau complexés, dans l'émulsion selon l'invention va de préférence de 52 à 90 % en poids, et mieux de 60 à 85 % en poids par rapport au poids total de la phase aqueuse à faible activité en eau.

On peut citer comme homopolymère complexant l'eau et les polyols, ceux vendus sous les dénominations de Norgel et de Lubrajel CG par la société Guardian. Ces polymères sont des polyacrylates de glycéryle complexés avec plus de 65 % de glycérine et/ou de propylène glycol et moins de 35 % en poids d'eau. Ces polymères apportent le polyol et l'eau complexés, et éventuellement jouent en outre le rôle de gélifiant de la composition.

Les actifs sensibles à l'eau qui peuvent être utilisés selon l'invention sont notamment les enzymes (par exemple lactoperoxydase, lipase, protéase, phospholipase, cellulases), les extraits naturels tels que thé vert, extrait de mélisse, extrait de thym, les oligomères procyannidoliques (OPC) tels que l'OPC d'aubépine, l'OPC de pin et l'OPC de raisin, les vitamines et notamment l'acide ascorbique (vitamine C) et ses esters, le rétinol (vitamine A) et ses esters, les dérivés phosphatés et glucosylés, l'urée et la rutine.

Le ou les actifs sensibles à l'eau utilisés sont de manière avantageuse une enzyme, et plus particulièrement une protéase. Cette protéase peut être choisie par exemple parmi celles vendues sous les dénominations commerciales《 Subtilisine SP 544 》, 《 Subtilisine SP 554 》, 《 Subtilisine SP 582 》 par la société Novo Nordisk et celle vendue sous la dénomination commerciale《 Lysoveg 》 par la société Laboratoires Sérobiologiques de Nancy.

La quantité d'actif sensible à l'eau dans la composition selon l'invention dépend du type d'actif utilisé. De manière générale, le ou les actifs peuvent être utilisés dans la composition selon l'invention en une quantité allant de 0,001 à 15 % en poids, de préférence de 0,01 à 10 %, et mieux de 0,05 à 5 % en poids par rapport au poids total de la composition.

Pour assurer l'émulsification de l'émulsion primaire dans la phase aqueuse externe continue de l'émulsion, celle-ci contient un polymère à caractère émulsionnant, notamment un polymère à chaîne grasse du type copolymère acide ou anhydride d'acide carboxylique monoéthylènique en $C_3$-$C_6$/ester acrylique à chaîne grasse. Ce polymère à chaîne grasse peut être choisi notamment parmi les copolymères commercialisés sous les dénominations 〈〈 Pemulen 〉〉 et 〈〈 Carbopol 1342 〉〉 ou 〈〈 Carbopol 1382 〉〉 par la société Goodrich. La quantité de tels polymères dans l'émulsion selon l'invention peut aller de préférence de 0,05 à 3 % en poids par rapport au poids total de l'émulsion.

La phase externe aqueuse continue peut en outre contenir un ou plusieurs gélifiants, par exemple un polymère carboxyvinylique comme ceux vendus sous les dénominations 〈〈 Carbopol 980 〉〉 ou 〈〈 Carbopol 942 〉〉 ou 〈〈 Carbopol 950 〉〉 par la société Goodrich ou celui vendu sous la dénomination 〈〈 Synthalen K 〉〉 par la société Sigma. La quantité de tels gélifiants dans l'émulsion selon l'invention peut aller de préférence de 0,05 à 3 % en poids par rapport au poids total de l'émulsion.

De façon avantageuse, la phase huileuse est essentiellement constituée par au moins un émulsionnant siliconé et une huile siliconée.

Les émulsionnants siliconés peuvent être choisis parmi les diméthicone copolyols et les alkyldiméthicone copolyols. On peut citer comme émulsionnant utilisable dans l'émulsion selon l'invention, le mélange de Polyglyceryl-4 isostearate/Cetyl dimethicone copolyol/Hexyl laurate vendu sous la dénomination 〈〈Abil WE 09 〉〉 par la société Goldschmidt, le Cetyl dimethicone copolyol vendu sous la dénomination 〈〈Abil EM 90 〉〉 par la société Goldschmidt et le mélange de cyclomethicone/dimethicone copolyol vendu sous la dénomination 〈〈Q2-3225C 〉〉 par la société Dow Corning. La quantité d'émulsionnant siliconé dans l'émulsion selon l'invention va de préférence de 0,05 à 10 % en poids et de préférence de 0,5 à 8 % en poids par rapport au poids total de l'émulsion triple.

Les huiles siliconées peuvent être choisies par exemple parmi les silicones volatiles telles que le cyclopentadiméthylsiloxane et le cyclotétradiméthylsiloxane, les polydiméthylsiloxanes, les polyphényltriméthylsiloxanes, les silicones fluorées. La quantité d'huiles siliconées va de 0,5 à 60 % en poids et de préférence de 10 à 30 % en poids par rapport au poids total de l'émulsion triple.

L'émulsion selon l'invention peut en outre comprendre un ou plusieurs autres corps gras choisi parmi les cires, les gommes ou les résines de silicone et éventuellement des huiles non siliconées. Ces corps gras peuvent être utilisés par exemple en une quantité allant de 0,05 à 5 % en poids par rapport au poids total de l'émulsion triple.

Comme cires, on peut utiliser notamment les cires de silicone telles que les alcoxydiméthylsiloxanes, et plus particulièrement les stéaroxypolydiméthylsiloxanes, les alkylpolysiloxanes et les polydiméthylsiloxanes à fonction mercapto.

Comme gommes, on peut utiliser notamment les gommes de silicone telles que les polydiméthylsiloxanes de haut poids moléculaire, les polydiméthylsiloxanes à terminaison hydroxyle (diméthiconols).

Comme résines, on peut utiliser notamment les résines de silicone telles que les triméthylsiloxysilicates.

Comme huiles non siliconées, on peut citer en particulier les huiles fluorées, les huiles d'origine animale ou végétale, les huiles minérales ou les huiles synthétiques.

L'émulsion primaire peut représenter par exemple de 10 à 40 % en poids par rapport au poids total de l'émulsion triple.

Selon un mode particulier de réalisation de l'invention, l'émulsion primaire constitue de 20 à 35 % et plus particulièrement 25 % en poids de l'émulsion triple, ce qui permet d'obtenir une émulsion triple translucide ou transparente.

L'émulsion triple est préparée de manière classique par préparation de l'émulsion primaire et incorporation d'une quantité déterminée de l'émulsion primaire dans la phase aqueuse externe.

Selon un mode particulier de réalisation de l'invention, l'émulsion primaire contient une partie de l'huile siliconée, par exemple au maximum 20 % en poids par rapport au poids total de l'émulsion primaire, et on ajoute ensuite le reste d'huile siliconée à la quantité d'émulsion primaire utilisée pour préparer l'émulsion triple, avant d'ajouter le mélange dans la phase aqueuse externe.

Pour une application topique, l'émulsion selon l'invention doit contenir un milieu topiquement acceptable, c'est-à-dire compatible avec la peau et les cheveux, et la composition à base de cette émulsion peut constituer notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

Aussi, l'invention a encore pour objet l'utilisation de la composition selon l'invention pour nettoyer et/ou protéger la peau et/ou les muqueuses et/ou les fibres kératiniques, c'est-à-dire les cheveux et/ou les cils.

L'invention a aussi pour objet un procédé cosmétique et/ou dermatologique pour nettoyer et/ou protéger la peau et/ou les muqueuses et/ou les fibres kératiniques, caractérisé en ce qu'il consiste à appliquer sur la peau et/ou les muqueuses et/ou les fibres kératiniques une composition telle que définie ci-dessus.

La présente invention a enfin pour objet une composition nettoyante pour la peau et/ou les fibres kératiniques, caractérisée en ce qu'elle contient une émulsion telle que définie ci-dessus et au moins un actif à action topique sensible à l'eau, contenu dans la phase aqueuse présentant une valeur d'activité en eau, inférieure ou égale à 0,85.

La composition selon l'invention peut constituer notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles en plus des actifs sensibles à l'eau, les conservateurs, les antioxydants, les solvants, les parfums, les charges, les filtres, les absorbeurs d'odeur, les matières colorantes et les vésicules lipidiques. Les quantités de ces différents adjuvants sont celles classiquement utilisées dans les domaines considérés, et par exemple de 0,01 % à 15 % du poids total de la composition. Ces adjuvants, selon leur nature, peuvent être introduits dans la phase grasse ou dans la phase aqueuse.

Comme actifs hydrophiles, on peut utiliser par exemple les protéines ou les hydrolysats de protéine, les acides aminés, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles.

Les exemples ci-après de compositions selon l'invention, sont donnés à titre d'illustration et sans caractère limitatif. Les quantités y sont données en % en poids.

## Exemple 1 : Emulsion triple pour le lissage du visage

| Emulsion primaire (E/H) : | |
| --- | --- |
| Phase huileuse : | |
| Abil WE-09 vendu par la société Goldschmidt | 2,5 % |
| Polydiméthylsiloxane | 4 % |
| Cyclopentadiméthylsiloxane | 17,5 % |
| Phase aqueuse : | |
| Sulfate de magnésium | 0,8 % |
| Conservateur | 0,2 % |
| Glycérine | 2 % |
| Propylène glycol | 20 % |
| Eau | 53 % |
| Emulsion triple : | |
| Emulsion primaire | 25 % |
| Cyclopentadiméthylsiloxane | 10 % |
| Subtilisine SP544 | 0,1 % |
| Carbopol 1342 | 0,3 % |
| Triéthanolamine | 0,3 % |
| Norgel à 67,8 % de glycérine | 52,7 % |
| Eau | 11,6 % |

Le mode opératoire pour préparer l'émulsion est le suivant : On prépare l'émulsion primaire en émulsionnant la phase aqueuse dans la phase huileuse par homogénéisation à la turbine. On prend 25 % de l'émulsion primaire obtenue et on y ajoute 10 % de cyclopentadiméthylsiloxane. Par ailleurs, on prépare la phase aqueuse externe en incorporant dans le Norgel, 10 % d'une solution aqueuse à 3 % de Carbopol 1342. Puis, on émulsifie l'émulsion primaire dans la phase aqueuse externe sous forte agitation ; on ajoute ensuite la triéthanolamine, et enfin la Subtilisine SP544.

L'activité en eau de la phase externe de cette émulsion est de $0{,}77 \pm 0{,}02$.

On obtient une crème transparente, apte à lisser et uniformiser la peau.

**Exemple 2 : Emulsion triple pour l'hydratation de la peau**

| Emulsion primaire (E/H) : | |
|---|---|
| Phase huileuse : | |
| Abil WE-09 vendu par la société Goldschmidt | 2,5 % |
| Polydiméthylsiloxane | 4 % |
| Cyclopentadiméthylsiloxane | 17,5 % |
| Phase aqueuse : | |
| Sulfate de magnésium | 0,8 % |
| Glycérine | 51 % |
| Propylène glycol | 20 % |
| Eau | 4,1 % |
| Subtilisine SP554 | 0,1 % |
| Emulsion triple : | |
| Emulsion primaire | 20 % |
| Cyclopentadiméthylsiloxane | 10 % |
| Gel de Carbopol 980 à 1 % en M.A. | 30 % |
| Conservateurs | 1 % |
| Carbopol 1382 | 0,3 % |
| Triéthanolamine | 0,3 % |
| Eau | 38,4 % |

Le mode opératoire pour préparer l'émulsion est le suivant : On prépare l'émulsion primaire en émulsionnant la phase aqueuse contenant la Subtilisine SP554, dans la phase huileuse par homogénéisation à la turbine. On ajoute à 20 % de l'émulsion primaire ainsi obtenue, 10 % de cyclopentadiméthylsiloxane. Par ailleurs, on prépare le gel de Carbopol 980 en mélangeant 1 % de Carbopol 980 avec 1 % de triéthanolamine et 98 % d'eau. On prépare la phase aqueuse externe en mélangeant ce gel avec le reste des constituants sauf la triéthanolamine. Puis, on émulsifie l'émulsion primaire dans la phase aqueuse externe sous forte agitation et on ajoute la triéthanolamine.

L'activité en eau de la phase aqueuse de l'émulsion primaire est de 0,22.

On obtient une crème blanche, apte à hydrater la peau.

On pourrait remplacer selon l'invention, dans les exemples ci-dessus, la Subtilisine par d'autres enzymes, l'acide ascorbique, le thé vert et les autres actifs sensibles à l'eau cités ci-dessus.

**Revendications**

1. Composition cosmétique et/ou dermatologique sous forme d'une émulsion triple eau/huile/eau comportant une phase aqueuse externe, une phase huileuse constituant avec une phase aqueuse interne une émulsion primaire eau/huile, caractérisée en ce que l'une des phases aqueuses a une valeur d'activité en eau, inférieure ou égale à 0,85 et en ce qu'elle contient au moins un actif à action topique sensible à l'eau dans la phase aqueuse ayant une valeur d'activité en eau inférieure ou égale à 0,85.

2. Composition selon la revendication 1, caractérisée en ce que la phase aqueuse ayant une valeur d'activité en eau, inférieure ou égale à 0,85 comprend une quantité efficace d'au moins un polyol.

3. Composition selon la revendication 1 ou 2, caractérisée en ce que la phase huileuse comprend au moins une huile siliconée et un émulsionnant siliconé.

4. Composition selon l'une quelconque des revendications précédentes, caractérisée en ce que la phase aqueuse ayant une activité en eau, inférieure ou égale à 0,85 est la phase aqueuse externe.

5. Composition selon l'une quelconque des revendications précédentes, caractérisée en ce que la phase aqueuse ayant une valeur d'activité en eau, inférieure ou égale à 0,85 comprend au moins un polyol en une quantité efficace pour obtenir une valeur d'activité en eau de la dite phase, inférieure ou égale à 0,75.

6. Composition selon l'une quelconque des revendications 2 à 5, caractérisée en ce que le polyol est présent dans la phase aqueuse ayant une activité en eau inférieure ou égale à 0,85, en une quantité allant de 35 à 90 % en poids par rapport au poids total de cette phase aqueuse.

7. Composition selon l'une quelconque des revendications 2 à 6, caractérisée en ce que le polyol est présent dans la phase aqueuse ayant une activité en eau inférieure ou égale à 0,85, en une quantité allant de 35 à 70 % en poids par rapport au poids total de l'émulsion.

8. Composition selon l'une quelconque des revendications 2 à 7, caractérisée en ce que le polyol est choisi dans le groupe comprenant la glycérine et les glycols.

9. Composition selon l'une quelconque des revendications 2 à 8, caractérisée en ce que le polyol est complexé avec un polymère acrylique ou méthacrylique.

10. Composition selon la revendication précédente, caractérisée en ce que le polymère comprend en outre de l'eau liée.

11. Composition selon la revendication 9 ou 10 , caractérisée en ce que le polymère avec le polyol et l'eau complexés est présent en une quantité allant de 52 à 90 % en poids par rapport au poids total de la phase aqueuse ayant une activité en eau, inférieure ou égale à 0,85.

12. Composition selon l'une quelconque des revendications précédentes, caractérisée en ce que la phase aqueuse externe de l'émulsion triple comprend un polymère à chaîne grasse.

13. Composition selon la revendication précédente, caractérisée en ce que le polymère à chaîne grasse est un copolymère acide ou anhydride d'acide carboxylique monoéthylènique en $C_3$-$C_6$/ester acrylique à chaîne grasse.

14. Composition selon la revendication 12 ou 13, caractérisée en ce que le polymère à chaîne grasse représente de 0,05 à 3 % en poids par rapport au poids total de l'émulsion.

15. Composition selon l'une quelconque des revendications précédentes, caractérisée en ce que la phase huileuse comprend au moins une huile siliconée en une quantité allant de 0,5 à 60 % en poids par rapport au poids total de l'émulsion triple.

16. Composition selon la revendication précédente, caractérisée en ce que l'huile siliconée est choisie dans le groupe comprenant les silicones volatiles, les polydiméthylsiloxanes, les polyphényltriméthylsiloxanes, les silicones fluorées.

17. Composition selon l'une quelconque des revendications précédentes, caractérisée en ce que l'émulsionnant est choisi parmi les alkyldiméthicone copolyols et les diméthicone copolyols.

18. Composition selon l'une quelconque des revendications précédentes, caractérisée en ce que l'émulsionnant est présent en une quantité allant de 0,05 à 10 % en poids par rapport au poids total de l'émulsion.

19. Composition selon l'une quelconque des revendications précédentes, caractérisée en ce que la phase huileuse contient en outre un ou plusieurs autres corps gras choisi parmi les cires, les gommes ou les résines de silicone et les huiles non siliconées.

20. Composition selon l'une quelconque des revendications précédentes, caractérisée en ce que l'émulsion primaire eau/huile représente 20 à 35 % en poids par rapport au poids total de l'émulsion.

21. Composition selon l'une quelconque des revendications précédentes, caractérisée en ce qu'elle est transparente.

22. Composition selon l'une quelconque des revendications précédentes, caractérisée en ce que l'actif à action topique sensible à l'eau est choisi dans le groupe comprenant les enzymes, les extraits naturels, les oligomères procyannidoliques, les vitamines, les dérivés phosphatés et glucosylés, l'urée et la rutine.

23. Composition selon l'une quelconque des revendications précédentes, caractérisée en ce que l'actif à action topique sensible à l'eau est choisi dans le groupe comprenant une protéase, le thé vert, l'acide ascorbique, la vitamine A.

24. Composition selon l'une quelconque des revendications précédentes, caractérisée en ce que l'actif sensible à l'eau est présent en une concentration allant de 0,001 à 15% en poids par rapport au poids total de l'émulsion.

25. Composition selon l'une quelconque des revendications précédentes, caractérisée en ce qu'elle comprend au moins un adjuvant lipophile ou hydrophile choisi parmi les conservateurs, les antioxydants, les parfums, les charges, les filtres, les séquestrants, les huiles essentielles, les matières colorantes, les actifs hydrophiles ou lipophiles, et les vésicules lipidiques.

26. Utilisation cosmétique de la composition selon l'une quelconque des revendications précédentes pour nettoyer et/ou protéger la peau et/ou les muqueuses et/ou les fibres kératiniques.

27. Procédé cosmétique pour nettoyer et/ou protéger la peau et/ou les muqueuses et/ou les fibres kératiniques, caractérisé en ce qu'il consiste à appliquer sur la peau et/ou les muqueuses et/ou les fibres kératiniques une composition selon l'une quelconque des revendications 1 à 25.

28. Composition selon l'une quelconque des revendications 1 à 25, caractérisée en ce qu'elle constitue une composition nettoyante pour la peau et/ou les fibres kératiniques.

## Claims

1. Cosmetic and/or dermatological composition in the form of a water/oil/water triple emulsion comprising an outer aqueous phase and an oily phase constituting, with an inner aqueous phase, a water/oil primary emulsion, characterized in that one of the aqueous phases has a water activity value of less than or equal to 0.85 and in that it comprises at least one water-sensitive active principle with a topical effect in the aqueous phase having a water activity value of less than or equal to 0.85.

2. Composition according to Claim 1, characterized in that the aqueous phase having a water activity value of less than or equal to 0.85 comprises an effective amount of at least one polyol.

3. Composition according to Claim 1 or 2, characterized in that the oily phase comprises at least one silicone oil and one silicone emulsifier.

4. Composition according to any one of the preceding claims, characterized in that the aqueous phase having a water activity of less than or equal to 0.85 is the outer aqueous phase.

5. Composition according to any one of the preceding claims, characterized in that the aqueous phase having a water activity value of less than or equal to 0.85 comprises at least one polyol in an amount which is effective in producing a water activity value of the said phase which is less than or equal to 0.75.

6. Composition according to any one of Claims 2 to 5, characterized in that the polyol is present in the aqueous phase having a water activity of less than or equal to 0.85 in an amount ranging from 35 to 90% by weight with respect to the total weight of this aqueous phase.

7. Composition according to any one of Claims 2 to 6, characterized in that the polyol is present in the aqueous phase having a water activity of less than or equal to 0.85 in an amount ranging from 35 to 70% by weight with respect to the total weight of the emulsion.

8. Composition according to any one of Claims 2 to 7, characterized in that the polyol is chosen from the group comprising glycerol and glycols.

9. Composition according to any one of Claims 2 to 8, characterized in that the polyol is complexed with an acrylic or methacrylic polymer.

10. Composition according to the preceding claim, characterized in that the polymer additionally comprises bound water.

11. Composition according to Claim 9 or 10, characterized in that the polymer with the complexed polyol and the complexed water is present in an amount ranging from 52 to 90% by weight with respect to the total weight of the aqueous phase having a water activity of less than or equal to 0.85.

12. Composition according to any one of the preceding claims, characterized in that the outer aqueous phase of the triple emulsion comprises a polymer with a fatty chain.

13. Composition according to the preceding claim, characterized in that the polymer with a fatty chain is a $C_3$-$C_6$ monoethylenic carboxylic acid anhydride or acid/acrylic ester with a fatty chain copolymer.

14. Composition according to Claim 12 or 13, characterized in that the polymer with a fatty chain represents from 0.05 to 3% by weight with respect to the total weight of the emulsion.

15. Composition according to any one of the preceding claims, characterized in that the oily phase comprises at least one silicone oil in an amount ranging from 0.5 to 60% by weight with respect to the total weight of the triple emulsion.

16. Composition according to the preceding claim, characterized in that the silicone oil is chosen from the group comprising volatile silicones, polydimethylsiloxanes, polyphenyltrimethylsiloxanes or fluorinated silicones.

17. Composition according to any one of the preceding claims, characterized in that the emulsifier is chosen from alkyldimethicone copolyols and dimethicone copolyols.

18. Composition according to any one of the preceding claims, characterized in that the emulsifier is present in an amount ranging from 0.05 to 10% by weight with respect to the total weight of the emulsion.

19. Composition according to any one of the preceding claims, characterized in that the oily phase additionally comprises one or a number of other fatty substances chosen from silicone waxes, gums or resins and non-silicone oils.

20. Composition according to any one of the preceding claims, characterized in that the water/oil primary emulsion represents 20 to 35% by weight with respect to the total weight of the emulsion.

21. Composition according to any one of the preceding claims, characterized in that it is transparent.

22. Composition according to any one of the preceding claims, characterized in that the water-sensitive active principle with a topical effect is chosen from the group comprising enzymes, natural extracts, procyanidol oligomers, vitamins, phosphated and glucosylated derivatives, urea and rutin.

23. Composition according to any one of the preceding claims, characterized in that the water-sensitive active principle with a topical effect is chosen from the group comprising a protease, green tea, ascorbic acid or vitamin A.

24. Composition according to any one of the preceding claims, characterized in that the water-sensitive active principle is present in a concentration ranging from 0.001 to 15% by weight with respect to the total weight of the emulsion.

25. Composition according to any one of the preceding claims, characterized in that it comprises at least one lipophilic or hydrophilic adjuvant chosen from preservatives, antioxidants, fragrances, fillers, screening agents, sequestering agents, essential oils, colouring materials, hydrophilic or lipophilic active principles and lipid vesicles.

26. Cosmetic use of the composition according to any one of the preceding claims for cleansing and/or protecting the skin and/or mucous membranes and/or keratinous fibres.

27. Cosmetic process for cleansing and/or protecting the skin and/or mucous membranes and/or keratinous fibres, characterized in that it comprises the application to the skin and/or mucous membranes and/or keratinous fibres of a composition according to any one of Claims 1 to 25.

28. Composition according to any one of Claims 1 to 25, characterized in that it constitutes a cleansing composition for the skin and/or keratinous fibres.

**Patentansprüche**

1. Kosmetische und/oder dermatologische Zusammensetzung in Form einer dreifachen Wasser/Öl/Wasser-Emulsion, die eine äußere wässerige Phase und eine Ölphase, die mit einer inneren wässerigen Phase eine Wasser/Öl-Primäremulsion bildet, aufweist, dadurch gekennzeichnet, daß eine der wässerigen Phasen einen Wert der Wasseraktivität von höchstens 0,85 aufweist, und dadurch, daß sie in der wässerigen Phase, die einen Wert der Wasseraktivität von höchstens 0,85 aufweist, mindestens einen gegenüber Wasser empfindlichen Wirkstoff mit topischer Wirkung enthält.

2. Zusammensetzung nach Anspruch 1, dadurch gekennzeichnet, daß die wässerige Phase, die einen Wert der Wasseraktivität von höchstens 0,85 aufweist, eine wirksame Menge mindestens eines Polyols enthält.

3. Zusammensetzung nach Anspruch 1 oder 2, dadurch gekennzeichnet, daß die Ölphase mindestens ein Siliconöl und mindestens einen siliconhaltigen Emulgator enthält.

4. Zusammensetzung nach einem der vorhergehenden Ansprüche, dadurch gekennzeichnet, daß die wässerige Phase, die eine Wasseraktivität von höchstens 0,85 aufweist, die äußere wässerige Phase ist.

5. Zusammensetzung nach einem der vorhergehenden Ansprüche, dadurch gekennzeichnet, daß die wässerige Phase, die einen Wert der Wasseraktivität von höchstens 0,85 aufweist, mindestens ein Polyol in einer wirksamen Menge enthält, um einen Wert der Wasseraktivität dieser Phase zu erzielen, der höchstens 0,75 beträgt.

6. Zusammensetzung nach einem der Ansprüche 2 bis 5, dadurch gekennzeichnet, daß das Polyol in der wässerigen Phase, die eine Wasseraktivität von höchstens 0,85 aufweist, in einem Mengenanteil im Bereich von 35 bis 90 Gew.-%, bezogen auf das Gesamtgewicht dieser wässerigen Phase, vorliegt.

7. Zusammensetzung nach einem der Ansprüche 2 bis 6, dadurch gekennzeichnet, daß das Polyol in der wässerigen Phase, die eine Wasseraktivität von höchstens 0,85 aufweist, in einem Mengenanteil im Bereich von 35 bis 70 Gew.-%, bezogen auf das Gesamtgewicht der Emulsion, vorliegt.

8. Zusammensetzung nach einem der Ansprüche 2 bis 7, dadurch gekennzeichnet, daß das Polyol unter Glycerin und Glykolen ausgewählt ist.

9. Zusammensetzung nach einem der Ansprüche 2 bis 8, dadurch gekennzeichnet, daß das Polyol mit einem Acrylpolymer oder einem Methacrylpolymer komplexiert ist.

10. Zusammensetzung nach dem vorhergehenden Anspruch, dadurch gekennzeichnet, daß das Polymer ferner gebundenes Wasser enthält.

11. Zusammensetzung nach Anspruch 9 oder 10, dadurch gekennzeichnet, daß das Polymer, das mit dem Polyol und dem Wasser komplexiert ist, in einem Mengenanteil im Bereich von 52 bis 90 Gew.-%, bezogen auf das Gesamtgewicht der wässerigen Phase, die eine Wasseraktivität von höchstens 0,85 aufweist, vorliegt.

12. Zusammensetzung nach einem der vorhergehenden Ansprüche, dadurch gekennzeichnet, daß die äußere wässerige Phase der dreifachen Emulsion ein Polymer mit Fettkette enthält.

13. Zusammensetzung nach dem vorhergehenden Anspruch, dadurch gekennzeichnet, daß das Polymer mit Fettkette ein Copolymer einer $C_{3-6}$-Carbonsäure mit einer ethylenisch ungesättigten Bindung oder eines Anhydrids einer $C_{3-6}$-Carbonsäure mit einer ethylenisch ungesättigten Bindung mit einem Acrylester mit Fettkette ist.

**14.** Zusammensetzung nach Anspruch 12 oder 13, dadurch gekennzeichnet, daß das Polymer mit Fettkette 0,05 bis 3 Gew.-%, bezogen auf das Gesamtgewicht der Emulsion, ausmacht.

**15.** Zusammensetzung nach einem der vorhergehenden Ansprüche, dadurch gekennzeichnet, daß die Ölphase mindestens ein Siliconöl in einem Mengenanteil im Bereich von 0,5 bis 60 Gew.-%, bezogen auf das Gesamtgewicht der dreifachen Emulsion, enthält.

**16.** Zusammensetzung nach dem vorhergehenden Anspruch, dadurch gekennzeichnet, daß das Siliconöl unter den flüchtigen Siliconen, Polydimethylsiloxanen, Polyphenyltrimethylsiloxanen und fluorierten Siliconen ausgewählt ist.

**17.** Zusammensetzung nach einem der vorhergehenden Ansprüche, dadurch gekennzeichnet, daß der Emulgator unter den Alkyldimeticoncopolyolen und Dimeticoncopolyolen ausgewählt ist.

**18.** Zusammensetzung nach einem der vorhergehenden Ansprüche, dadurch gekennzeichnet, daß der Emulgator in einem Mengenanteil im Bereich von 0,05 bis 10 Gew.-%, bezogen auf das Gesamtgewicht der Emulsion, vorliegt.

**19.** Zusammensetzung nach einem der vorhergehenden Ansprüche, dadurch gekennzeichnet, daß die Ölphase ferner eine oder mehrere weitere Fettsubstanzen enthält, die unter Siliconwachsen, Silicongummis oder Siliconharzen und nicht siliconhaltigen Ölen ausgewählt sind.

**20.** Zusammensetzung nach einem der vorhergehenden Ansprüche, dadurch gekennzeichnet, daß die Wasser/Öl-Primäremulsion 20 bis 35 Gew.-%, bezogen auf das Gesamtgewicht der Emulsion, ausmacht.

**21.** Zusammensetzung nach einem der vorhergehenden Ansprüche, dadurch gekennzeichnet, daß sie durchsichtig ist.

**22.** Zusammensetzung nach einem der vorhergehenden Ansprüche, dadurch gekennzeichnet, daß der gegenüber Wasser empfindliche Wirkstoff mit topischer Wirkung unter Enzymen, natürlichen Extrakten, Procyanidololigomeren, Vitaminen, phosphatierten und glucosylierten Derivaten, Harnstoff und Rutin ausgewählt ist.

**23.** Zusammensetzung nach einem der vorhergehenden Ansprüche, dadurch gekennzeichnet, daß der gegenüber Wasser empfindliche Wirkstoff mit topischer Wirkung unter einer Protease, grünem Tee, Ascorbinsäure und Vitamin A ausgewählt ist.

**24.** Zusammensetzung nach einem der vorhergehenden Ansprüche, dadurch gekennzeichnet, daß der gegenüber Wasser empfindliche Wirkstoff in einer Konzentration im Bereich von 0,001 bis 15 Gew.-%, bezogen auf das Gesamtgewicht der Emulsion, vorliegt.

**25.** Zusammensetzung nach einem der Vorhergehenden Ansprüche, dadurch gekennzeichnet, daß sie mindestens einen lipophilen oder hydrophilen Zusatzstoff enthält, der unter Konservierungsmitteln, Antioxidantien, Parfums, Füllstoffen, Filtern, Maskierungsmitteln, etherischen Ölen, Färbemitteln, hydrophilen oder lipophilen Wirkstoffen und Lipidvesikeln ausgewählt ist.

**26.** Kosmetische Verwendung der Zusammensetzung nach einem der vorhergehenden Ansprüche zur Reinigung und/oder zum Schutz der Haut und/oder der Schleimhäute und/oder der Keratinfasern.

**27.** Kosmetisches Verfahren zur Reinigung und/oder zum Schutz der Haut und/oder der Schleimhäute und/oder der Keratinfasern, dadurch gekennzeichnet, daß es darin besteht, eine Zusammensetzung nach einem der Ansprüche 1 bis 25 auf die Haut und/oder die Schleimhäute und/oder die Keratinfasern aufzutragen.

**28.** Zusammensetzung nach einem der Ansprüche 1 bis 25, dadurch gekennzeichnet, daß sie eine reinigende Zusammensetzung für die Haut und/oder die Keratinfasern darstellt.